(19) Europäisches Patentamt
European Patent Office
Office européen des brevets

(11) Publication number: **0 464 388 A1**

(12) **EUROPEAN PATENT APPLICATION**

(21) Application number: **91109247.6**

(22) Date of filing: **06.06.91**

(51) Int. Cl.$^5$: **C12Q 1/00**, C12Q 1/46, //C12Q1/37,C12Q1/32, C12Q1/34

(30) Priority: **18.06.90 US 539654**

(43) Date of publication of application: **08.01.92 Bulletin 92/02**

(84) Designated Contracting States: **BE CH DE DK ES FR GB IT LI NL SE**

(71) Applicant: **TECHNICON INSTRUMENTS CORPORATION (a New York corporation)**
**511 Benedict Avenue**
**Tarrytown New York 10591-5097(US)**

(72) Inventor: **Hatch, Robert O.**
**1807 Filbert Way**
**Elkhart, Indiana 46514(US)**
Inventor: **Vlastelica, Daniel L.**
**34 Esther Avenue**
**Congers, New York 10920(US)**
Inventor: **Yun, Shyon-Long**
**29 Glendale Avenue**
**Armonk New York 10504(US)**

(74) Representative: **Dänner, Klaus, Dr. et al**
**c/o Bayer AG Konzernverwaltung RP Patente Konzern**
**W-5090 Leverkusen 1 Bayerwerk(DE)**

(54) **Detection assays having chromogenic and nonchromogenic, hypocromogenic, bathochromogenic or hypsochromogenic substrates.**

(57) The invention relates to enzyme detection assay compositions and methods which have chromogenic and nonchromogenic, hypochromogenic, bathochromogenic or hypsochromogenic substrates. By using both chromogenic and nonchromogenic, hypochromogenic, bathochromogenic or hypsochromogenic substrates assay sensitivity can be controlled and the need for sample predilution eliminated. In one embodiment of the invention an assay for the estimation of serum cholinesterase activity is provided.

EP 0 464 388 A1

## Field Of The Invention

This invention relates to means for regulating enzyme activity using a competitive substrate and, more particularly, to enzyme detection assay compositions and methods which have or use chromogenic and nonchromogenic, hypochromogenic, bathochromogenic or hypsochromogenic substrates.

## Background Of The Invention

The present invention has broad application to chemical systems in which enzyme activity is regulated using a competitive substrate. The invention is particularly applicable to novel detection assays employing both chromogenic and nonchromogenic, hypochromogenic, bathochromogenic or hypsochromogenic substrates.

A chromogenic substrate is a substrate which absorbs light strongly in the visible region. A nonchromogenic substrate is a substrate which does not absorb light in the visible region. A hypochromogenic substrate is a substrate which absorbs light only weakly in the visible region (i.e., absorbs less than 10% light in the visible region compared to the chromogenic substrate). A bathochromogenic substrate is a substrate which absorbs light in the visible region at a higher wavelength compared to another substrate (i.e., a chromogenic substrate). Finally, a hypsochromogenic substrate is a substrate which absorbs light in the visible region at a lower wavelength compared to another substrate (i.e., a chromogenic substrate).

Such systems can be illustrated by, but are not limited to, an assay for cholinesterase in which chromogenic and nonchromogenic substrates are used to control sensitivity of the assay and therefore eliminate the need for sample predilution. Estimation of serum cholinesterase activity is of proven value in industrial or agricultural toxicology, in biochemical genetics and in screening of patients who are abnormally sensitive to succinyldicholine.

Cholinesterase is an enzyme which hydrolyses esters of choline. This enzyme appears to be formed in the liver and the concentration of cholinesterase appearing in plasma at any one time is a reflection of the rate of formation of the enzyme by the liver. In the absence of known inhibitors, any decrease in activity in serum reflects impaired synthesis of the enzyme by the liver. A 30% to 50% decrease in the level of cholinesterase is observed in acute hepatitis and in chronic hepatitis of long duration. Decreases of 50% to 70% in the level of cholinesterase occur in advanced cirrhosis and carcinoma with metastases to the liver. Thus, the determination of the enzyme may be taken as a sensitivity test of liver function.

In addition, serum cholinesterase is irreversibly inhibited by organophosphates and carbamates which are highly toxic. Organophosphates are widely used insecticides, in agricultural and horticultural practice. For example, Parathion and Malathion contain organophosphates. Ingestion of food contaminated with insecticides is a common cause of organophosphorous poisoning. In countries such as Japan, where insecticides are widely used to protect rice crops and where crop spraying pilots of necessity perform their spraying at low levels, there is considerable risk of inhalation of concentrated amounts of insecticides.

The systemic affect of acute poisoning becomes apparent within an hour or two and may last for several days, depending on dose and the route of absorption - either through the skin, respiratory tract, conjuctivae or alimentary tract. Chronic poisoning, in the generally accepted sense, does not occur, but continual absorption of subtoxic doses can occur in agricultural workers, finally accumulating in the features of acute poisoning and even death.

Certainly, continual exposure to subtoxic doses of organophosphates diminishes the activity of serum cholinesterase. When the exposure ceases cholinesterase activities revert to normal over a period of five to six weeks as the enzyme is resynthesized by the liver. For this reason an assay of serum cholinesterase is preferred for screening and monitoring agricultural and industrial workers since it is capable of good definition.

Serum cholinesterase is also assayed in patients who exhibit sensitivity to suxamethonium, which is marketed in the United Kingdom as Scoline. This is a widely used, short acting muscle relaxant first introduced into clinical anesthesia in 1951.

Numerous procedures are available for the estimation of serum cholinesterase. Because of its relative simplicity and sensitivity an assay based on the hydrolysis of thiocholine esters is preferred. To determine enzyme activity, either by manual or automated means, requires that that activity fall within a preset linearity range. This is limited by either instrument capabilities or concentration of the substrate in the reaction mixture. If the sample activity is beyond a preset range, a smaller amount of sample must be used and/or it must be prediluted. In many clinical laboratories, where hundreds of samples are assayed each day, predilution becomes tedious and time-consuming and contributes to assay imprecision. Furthermore, it increases the risk of exposure to biohazardous materials. Moreover, adjusting the sample volume is not

always feasible for those automated analyzers which utilize a fixed ratio of sample to reagent.

In the course of the development of an assay for cholinesterase in serum the problem of excess activity was uncovered particularly when attempts were made to adapt the assay to the TECHNICON CHEM 1® analyzer. A procedure to overcome this problem without predilution was needed.

Due to the extreme sensitivity of the assay, ways were first sought to make the test less sensitive without requiring predilution. Conventional approaches, involving changes in the buffer, were attempted without success. In addition, the use of other substrates only appeared to complicate the procedure.

In accordance with the present invention a system for controlling assay sensitivity was discovered thereby eliminating the need for sample predilution.

## Summary Of The Invention

An object of the present invention is to provide means for regulating enzyme activity using a competitive substrate.

Another object of the present invention is provide means for regulating enzyme detection assays by using both chromogenic and nonchromogenic, hypochromogenic, bathochromogenic or hypsochromogenic substrates.

Still another object of the present invention is to provide novel compositions and methods for determining cholinesterase activity.

Yet another object of the present invention is to provide compositions and methods for determining cholinesterase activity which do not require predilution of the sample.

A further object of the present invention is to provide an enzyme assay method which eliminates the necessity for predilution of a sample while providing an accurate and simple determination having high sensitivity.

In accordance with the present invention it was discovered that by employing at least two substrates, one of which is chromogenic and at least one of which is nonchromogenic, hypochromogenic, bathochromogenic or hypsochromogenic, that it is possible to provide an enzyme assay method of high accuracy and sensitivity which does not require predilution of the sample.

The invention can be illustrated by, but is not limited to, the determination of cholinesterase using butyrylcholine and butyrylthiocholine as dual substrates. Cholinesterase hydrolyzes both butyrylcholine and butyrylthiocholine. However, since the reaction mixture contains two substrates, one of them being chromogenic and the other nonchromogenic, the signal resulting from the assay is smaller compared to a reaction mixture containing only a single chromogenic substrate. Thus, in the case of cholinesterase, both substrates are hydrolyzed but only thiocholine is detected. By adjusting the ratio of butyrylcholine to butyrylthiocholine, the output response can be controlled. By using two or more substrates, the concentration of the nonchromogenic substrate(s) can be increased to extend linearity without increasing the signal.

Thus, it is possible to provide enzyme detection assays which avoid the necessity of predilution. The invention serves as a replacement for traditional methods requiring predilution and provides a way of assaying those enzymes which are susceptible to denaturation by high sample dilution.

## Brief Description Of The Drawing

Other and further objects, advantages and features of the invention will be apparent to those skilled in the art from the following detailed description thereof, taken in conjunction with the accompanying drawing in which:

Fig. 1 is a chart showing the correlation of one embodiment of the present invention to a standard method.

## Description Of The Preferred Embodiments

Traditionally, assays for the measurement of serum cholinesterase employ a single substrate (e.g.,butyrylthiocholine, acetylthiocholine, etc.) which is converted to thiocholine in the serum cholinesterase catalyzed reaction. The thiocholine so produced is then reacted with a chromogen or reagent (e.g., 5,5-dithio-bis-2-nitrobenzoic acid, DTNB) to develop color for measurement. However, the high serum cholinesterase activity in normal human serum generates extremely high absorbance on commercial instruments with a sample to reagent ratio of 1 to 14. The problem was to achieve a method which would use sample serums directly with a fixed sample to reagent ratio without sample predilution.

A solution to this problem involved the use of two substrates, butyrylthiocholine and butyrylcholine.

Although both substrates can be hydrolyzed by cholinesterase, only one of the enzymatic reaction products, namely thiocholine, can react with DTNB to form a product with measurable absorbance at 405 nanometers (nm). This permits commercial apparatus to be used to perform an assay for human serum cholinesterase with a sample to reagent ratio of 1 to 14 without having to perform any sample predilution step.

In the following material certain abbreviations or designations are used. The meaning of these abbreviations and designations are as follows:

| Abbreviation | Chemical Name |
|---|---|
| BTC | Butyrylthiocholine |
| PTC | Propionylthiocholine |
| ATC | Acetylthiocholine |
| BC | Butyrylcholine |
| DTNB | 5,5'-Dithiobis(2-nitrobenzoic acid) |
| TNB | 5-Thio-2-nitrobenzoic acid |
| EDTA | Ethylenediaminetetraacetic acid |
| TRIS | Tris(hydroxymethyl)aminomethane |
| CHE | Cholinesterase |
| Dextran T40 | Linear chains of α(1-6)-linked D-glucopyranosyl residues with branches due to α(1-3) linkages having an average molecular weight of 40,000. |
| Triton® X-100 | Triton® X-100 is a biodegradable liquid anhydrous 100% active nonionic surface-active agent from Rohm & Haas Co. It is a water soluble octylphenoxypolyethoxy-ethanol containing an average of 10 moles of ethylene oxide. |

The principle of the method is illustrated by the following equations:

$$BTC + H_2O \xrightarrow{CHE} \text{Butyrate + Thiochloine}$$

$$\xrightarrow{DTNB} \text{5-thio-2-nitrobenzoate (TNB) (absorbance at 405 nanometers)}$$

$$BC + H_2O \xrightarrow{CHE} \text{Butyrate + Choline}$$

$$\xrightarrow{DTNB} \text{(no reaction)}$$

The rate of change, in absorbance at 405 nanometers (nm), is thus directly proportional to the CHE activity when both BC and BTC are present. Sensitivity is 0.000136 Abs/min/U/L when read in a 1.5 mm pathlength cell (reference method unit).

In the assay of the present invention the essential materials are the chromogenic and nonchromogenic, hypochromogenic or hypsochromogenic substrates, a chromogen and a buffer. These materials constitute the composition to which the sample is added.

Any substrate which will be converted to thiocholine in the catalyzed reaction for the determination of cholinesterase can be used as the chromogenic substrate. Such substrates include those already listed above and as well as propionylthiocholine and acetylthiodidine. The preferred chromogenic substrate is butyrylthiocholine.

The preferred nonchromogenic substrate is butyrylcholine. Other suitable nonchromogenic substrates include acetylcholine and propionylthiocholine. As previously indicated, more than one nonchromogenic substrate can be present. For most assays, however, including assays for the determination of cholinesterase, no real advantage is obtained by using more than one nonchromogenic substrate.

The essential consideration in the described assays for the determination of cholinesterase is that both chromogenic and nonchromogenic substrates are present and that the chromogenic substrate be converted to thiocholine in reaction with cholinesterase. The preferred substrate materials are those which are relatively inexpensive and easily adapted for automation. The substrates should also be reasonably stable and must not interfere with the overall reaction. The ratio of the two subtrates is not critical and can be varied to the meet the requirements of the overall system.

The chromogen can vary in the final reaction mixture from about 0.2 to about 0.7mM (millimolar) with a preferred level at about 1 mM. The preferred chromogen is DTNB. Excessive DTNB will inhibit CHE activity whereas insufficient DTNB limits the range of linearity. Other suitable chromogens for the CHE reaction which can be used including DTBP [2,2'-dithiobis-pyridine], DTNP [2,2'-dithiobis-(5-nitropyridine)], DTNA [6,6'-dithiobis(nicotinic acid)], DSNB [6,6'-diselenobis-(3-nitrobenzoic acid)], 2,2'-dithiodibenzoic acid and 4,4'-dithiodipyridine, and the like.

When alternative chromogens are used (and particularly for systems directed to the determination of materials other than cholinesterase) the sensitivity, solubility, pH and optimum wavelength for such systems may change. For example, for DTNB a wavelength of ~ 410 nm and a pH of 7-8 would be used; for DSNB a wavelength of ~ 430 nm and a pH of 8 to 12 would be used; for DTNP a wavelength of ~ 385 and a pH of 5 to 9 would be used; for DTNA a wavelength of ~ 355 nm would be used; for 2,2'-dithiodipyridine a wavelength of ~ 340 nm and a pH of 4 to 5 would be used; and for 4,4'-dithiodipyridine a wavelength of ~ 325 nm would be used.

Any suitable buffer can be used for the reaction provided a proper pH is maintained and the buffer does not interfere with the overall reaction. Suitable buffers include tris amine, phosphate, two of the Goods buffers, HEPES [N-2-hydroxyethylpiperazine-N'-2-ethanesulfonicacid], MOPS [2(N-morpholino)-propanesulfonic acid], and TES [N-tris(hydroxymethyl)methyl-2aminoethanesulfonic acid]. The concentration of the buffer is not critical provided the pH is maintained. The concentration of the buffer will normally range from about 50 to about 200 mM.

The pH will depend on the other materials present in the assay system. For assays directed to the estimation of serum cholinesterase activity the pH will normally be in the range of about 4 to about 11 with a pH of about 7 to about 8.5 being particularly preferred. The optimum pH range for serum cholinesterase is between about 8 and about 8.5. For some substrates pH values above about 8 could cause the substrate to hydrolyze too rapidly.

The sample which is tested can be a body fluid such as whole blood, serum or plasma. When using the reagent systems of the present invention, any suitable reaction temperatures can be employed although the reaction temperatures normally used for ordinary clinical reactions are preferred. For example, standard temperatures, such as 25°C, 30°C or 37°C, are used and preferably the temperature of the assay is maintained at about 37°C. Absorbance measurements are read normally at 405 or 412 nanometers, but this depends on the particular chromogen used and the instrument.

In addition to the essential components listed above optional ingredients can be added. For example, chelating agents, such as EDTA, can be added to improve long term stability and prevent copper interference from carry-over of total protein biuretreagent into CHE as found on various chemistry analyzers. The concentration of the EDTA can range from about 0.2 to about 2 mM for chelation of heavy metals. Other suitable chelating agents which can be used include HEDTA (hydroxyethylethylenediaminetriacetic acid) and CDTA (cyclohexane-1,2-diaminetetraacetic acid).

In addition to a chelating agent, if desired, preservatives, surfactants and the like can be added in normal concentration levels for such materials.

In the formulation and use of the ingredients typically the sample is introduced and mixed with chromogen and buffer. The chromogenic and nonchromogenic substrates are then added to the reaction mixture. The absorbance of the resulting admixture is then read.

While the invention has been illustrated by reference to an assay for the determination of cholinesterase activity the invention is broadly directed to enzyme detection assay compositions and methods which have chromogenic and nonchromogenic, hypochromogenic, bathochromogenic or hypsochromogenic substrates. Examples of enzyme detection assay systems include alkaline phosphatase (EC 3.1.3.1.) and γ-glutamyl-transferase (EC 2.3.22). Basically, any enzyme can be detected using the present invention provided a nonchromogenic, hypochromogenic, bathochromogenic or hypsochromogenic competitive substrate is present.

Other examples of the use of substrates to reduce the extinction coefficient of a more bathochromogenic substrate are as follows. The color reduction effect will vary with the differences in the $K_m$ and $V_{max}$ of the substrates with the enzyme. Hydrolytic enzymes, for example, such as chymotrypsin and elastase will cleave a mixture of 4-nitrophenyl ester or anilide derivatives of a peptide to give a yellow colored product ($\lambda_{max}$ = 400-405 nm). These substrates can be used as competitors with a more bathochromic color producing system. For example, 4-nitrophenyl N-carbobenzyloxy-phenylalaninamide with chymotrypsin and 4-nitrophenyl N-tosylalaninate with elastase give enzyme cleavage products which do not couple with diazonium salts such as 4-diazo-2-naphthol-4-sulfonic acid and 2-methoxy-4-morpholinobenzenediazonium chloride (zinc chloride) double salt. However, the indoxyl and 2-hydroxy-5-phenylpyrrolylesters and 3-aminoindoxyl amide derivatives of these peptides will couple with the diazonium salts and give a bathochromic color (usually magenta, $\lambda_{max}$ = 520 nm). The color rate assay will be lower in the presence of the non- or hypsochromogenic substrates.

Substrates of other hydrolytic enzymes, such as γ-glutamyl transferase, will behave similarly.

The invention will be further illustrated by means of the following examples, to which the invention is not limited.

## EXAMPLE I

Seventy-eight human serum samples were assayed using a TECHNICON CHEM 1® analyzer with a temperature of 37° C using two substrates, BTC and BC, in accordance with the present invention. Identical samples, after a 71 fold dilution with phosphate buffer, were also assayed on a TECHNICON CHEM 1® analyzer using the ACB (Association of Clinical Biochemist) method with BTC as the single substrate. For the ACB method the conditions employed were as described in King and Whittacker, Ann. Clin. Biochem., 16:57-75.

The specimen population consisted of the following samples: 50 normal, random sera, 5 lipemic, 5 icteric, 5 hemolyzed, 5 uremic sera and 8 other seras spiked with human serum CHE.

The formulation for the tests performed in accordance with the invention included the following:

| Reagent 1 (Lyophilized Component) | |
|---|---|
| Raw Materials | Quantity Per Ampule |
| DTNB | 3.75 U mole |
| Dextran T40 | 24 mg (milligrams) |
| Tris | 8.92 U mole |
| Gentamicin Sulfate | 0.2 mg |

| Diluent 1 (Liquid Component) | |
|---|---|
| Raw Materials | Quantity Per Ampule |
| Tris | 400 U mole |
| EDTA | 8 U mole |
| Triton® X-100 | 4 mg |

| Reagent 2 (Lyophilized Component) | |
|---|---|
| Raw Materials | Quantity Per Ampule |
| BTC<br>BC<br>Gentamicin Sulfate | 48 U mole<br>1600 U mole<br>0.4 mg |

| Diluent 2 Liquid Component | |
|---|---|
| Raw Material | Quantity Per Ampule |
| P-hydroxybenzoic Acid<br>Triton® X-100<br>Gentamicin Sulfate | 4 mg<br>4 mg<br>0.4 mg |

The final reaction concentrations were as follows:

| Constituent | Concentration |
|---|---|
| DTNB | 0.44 mM |
| EDTA | 0.93 mM |
| Tris | 47.7 mM |
| BTC | 5.6 mM |
| BC | 186.7 mM |
| Dextran T40 | 0.28% |
| Triton® X-100 | 0.093% |
| P-hydroxybenzoic Acid | 0.047% |
| Gentamicin Sulfate | 0.012% |
| pH | 7.4 |

The p-hydroxy benzoate and gentamicin sulfate are used to prevent microbial growth. The Dextran T40 is a filler.

Actual sensitivity at both 30°C and 37°C was 0.000136 Abs/min/U/L in a 0.15 cm (centimeter) pathlength. Measurements were made at 405 nm. The compositions were stable for at least 30 days at 4°C.

The within-run, and total precisions are shown below.

| Sample U/L | CV Percentage Within Run | Total |
|---|---|---|
| 790 | 0.7 | 0.7 |
| 238 | 0.6 | 0.7 |
| 106 | 0.9 | 1.3 |
| 408 | 0.6 | 0.8 |

The correlation of the resulting method (the "CHEM 1" method) compared with standard ACB method is illustrated in Fig. 1. With all samples included in the linear regression analysis the following correlation results were obtained.

$$Y = 0.0825 x - 8.81$$

As seen in Fig. 1, the CHEM1 method of the present invention is linear to the ACB method.

As indicated previously, the present invention is applicable to detection assays employing both chromogenic and nonchromogenic, hypochromogenic, bathochromogenic or hypsochromogenic substrates

for enzymes other than cholinesterase. Representative of this are the following examples.

**EXAMPLE II**

Reductive Systems-Disulfide Indicators

Glutathione and lipoamide are reduced by their respective dehydrogenases to give thiols which can be detected using a chromogenic indicator such as DTNB or IBTZ [3-N-(3-dimethylaminopropyl)-5-nitroisobenzothiazol-3-one]. This example demonstrates that the addition of a nonchromogenic indicator or a yellow-producing indicator will reduce the amount of color expected from the reaction of a thiol with the bathochromic thiol indicator. These indicators (substrates) will reduce with reduced nicotinamide adenine dinucleotide (NADH) and the aforementioned enzymes. If the concentration of NADH is kept constant, the rate of color formation will be proportional to the enzyme concentration.

In addition, NADH is formed by the action of dehydrogenases, such as glucose or cholesterol dehydrogenase, on glucose or cholesterol. Consequently this provides a method of detecting these dehydrogenases by keeping the concentration of NAD, lipoamide or glutathione dehydrogenase, and substrate (for example, glucose or cholesterol) constant and detecting the rate of color formation.

To a cuvette containing a mixture of 85 nanomoles (nmoles) of the chromogenic thiol indicator N-methyl-5-(1-hydroxy-3,6-disulfo-2-napthylazo)benzoisothiazolone and 85 nanomoles of the nonchromogenic or hypsochromic indicators A, B or C (as set forth below) in 2.9 ml (milliliters) of 0.1 M (molar) phosphate, pH 6.5, was added to increasing amounts of 0.6 M dithiothreitol (DTT). The contents were mixed by inversion and the absorbance at 610 nm after two minutes was determined as listed below:

**Nonchromogenic Materials Evaluated**

A N-(3-dimethylaminopropyl)-5-acetamidobenzoisothiazolone
B N-(3-dimethylaminopropyl)-5-aminobenzoisothiazolone
C N-(3-dimethylaminopropyl)-5-nitrobenzoisothiazolone

| Nonchromogenic Reactant | nmoles of DTT | Absorbance at 610 nm |
|---|---|---|
| None | 0 | 0.002 |
| | 39 | 0.118 |
| | 74 | 0.224 |
| | 118 | 0.265 |
| A | 79 | 0.033 |
| | 158 | 0.134 |
| | 238 | 0.241 |
| B | 79 | 0.019 |
| | 158 | 0.153 |
| | 238 | 0.232 |
| C | 79 | 0.008 |
| | 158 | 0.90 |
| | 238 | 0.250 |

The result clearly indicate that the use of the non- or hypsochromogenic indicators reduce the color generated by the more bathochromic indicator.

(Buffer, as set forth in the following material, refers to 0.1 M, pH 6.9 N-(2-acetamido)-2-aminoethanesulfonic acid)

Compositions:

1. 0.0209 mmol/L N-(3-dimethylaminopropyl)-5-(1-hydroxy-3,6-disulfo-2-naphthyl)azoisobenzothiazol-3-one (IBTZ1, chromogenic indicator) in buffer,

2. 0.209 mmol/L IBTZ1, 0.219 mmol/L 5-aminoisobenzothiazol-3-one (IBTZ2, nonchromogenic indicator) in buffer,
3. 1.5 M glucose in water.
4. 6 mmol/L NAD in buffer.
5. 15.7 mmol/L lipoamide in methanol.
6. 20 U/mL of lipoamide dehydrogenase in 0.5% albumin in buffer.
7. freshly prepared 23.8 U/mL glucose dehydrogenase in 0.5% albumin in buffer.

A mixture of 0.5 mL of either Composition 1 (0.105 µmole) or Composition 2 (0.105 µmole IBTZ1, 0.110 µmole IBTZ2) was diluted with 2.05 mL of buffer. Compositions 3 (0.2 mL, 0.3 mmol), 4 (0.1 mL, 0.6 µmole), 5 (0.1 mL, 1.57 µmole), and 6 (0.050 mL, 1.0 U) were added. Quantities of 0.010, 0.020, 0.030, 0.040, and 0.050 mL of Composition 7 were added in separate experiments and the absorbance read at 614 nm after 45 seconds.

| Solution I | Absorbance | Solution II | Absorbance |
|---|---|---|---|
| 0.020 mL | 0.046 | 0.020 mL | 0.032 |
| 0.020 | 0.143 | | |
| 0.030 | 0.237 | 0.030 | 0.062 |
| 0.040 | 0.288 | | |
| 0.050 | 0.312 | 0.050 | 0.100 |

This data confirms that IBTZs can detect enzymes. The first column of data illustrates the detection of glucose dehydrogenase (GDH) by an IBTZ and the second shows how the addition of a non-chromogenic IBTZ suppresses the optical signal.

## EXAMPLE III

*β-Galactosidase*

The signal of a chromogenic substrate such as 7-β-galactopyranosyloxy-9,9-dimethyl-9H-acridinone ($\lambda_{max}$ = 632 nm) can be reduced by addition of either β-phenyl galactoside or β-2-(nitrophenyl) galactoside ($\lambda_{max}$ = 405 nm). The rate of color formation will be proportional to the amount of enzyme.

To 800 µL of assay stock solution (see below) in a UV cuvette cell is added 100 microliters (µL) of 50 mM of nonchromogenic, hypochromogenic or hypsochromogenic indicator (5 mM final concentration) or 100 µL of assay stock solution to determine the blank reading value. Then, 20 µL of 5 µg/mL horseradish peroxidase solution is added, timing is initiated, and the covered cuvette is shaken by inversion. The absorbance at 630 nm is monitored from 5 to 300 seconds.

| Assay Reagent | Volume (µL) | Final Assay Concentration |
|---|---|---|
| 1M Citrate, pH 4.5 | 200 | 0.2 M |
| o-tolidine, 20 mM | 100 | 5.0 mM |
| $H_2O_2$ | 100 | 0.2 mM |
| $H_2O$ | 480 | -- |
| Scavenger compound, 20 mM | 100 | 5.0 mM |
| Horseradish peroxidase, 5 µg/mL | 20 | 0.1 µg/dL |

Indicators for the scavenger include:

| Indicator | Initial Color | Oxidized Color |
|---|---|---|
| Acid Red #1 | orange | pale yellow |
| Acid Yellow #34 | yellow | colorless |
| Acid Orange #8 | orange | weak yellow |

Similar results are obtained for each indicator. The results set forth below are for Acid Red #1.

| Concentration of Nonchromogenic Indicator (mM) | Absorbance at 630 nm after 300 sec. |
|---|---|
| 0 | 1.067 |
| 5 | .530 |

The rate of color formation will be proportional to the amount of enzyme present in the initial sample.

From the foregoing, it will be seen that this invention is well adapted to attain all of the ends and objects hereinbefore set forth, together with other advantages which are obvious and inherent. Not only does the present invention eliminate the necessity for predilution but it provides an accurate and simple determination of enzyme activity. The method has high sensitivity. In addition to the other advantages, the present invention allows increased sample size for improved pipetting precision, prevents enzyme denaturation due to high dilution and permits an extended linear range.

Obviously, many modifications and variations of the invention as hereinbefore set forth can be made without departing from the spirit and scope thereof and therefore only such limitations should be imposed as are indicated by the appended claims.

**Claims**

1. A composition for the determination of enzyme activity in a sample, said composition comprising chromogenic and nonchromogenic, hypochromogenic, bathochromogenic or hypsochromogenic substrates, buffer and chromogen.

2. The composition of claim 1 in which the enzyme is cholinesterase.

3. The composition of claim 2 in which the chromogenic substrate is propionylthiocholine.

4. The composition of any of claims 2 and 3 in which butyrylcholine and butyrylthiocholine are present as substrates.

5. The composition of any of claims 2 to 4 in which the chromogen is selected from the group 5,5-dithiobis-2-nitrobenzoic acid, 2,2'-dithiobis-pyridine, 2,2'-dithiobis-(5-nitropyridine), 6,6'-dithiobis(nicotinic acid), 6,6'-diselenobis(3-nitrobenzoic acid), 2,2'-dithiodibenzoic acid and 4,4'-dithiodipyridine.

6. The composition of any of claims 1 to 5 in which the chromogen is present in a concentration of from about 0.2 to about 0.7 millimolar.

7. The composition of any of claims 1 to 6 in which the pH is maintained in the range of about 7 to about 8.5.

8. The composition of any of claims 1 to 7 in which the buffer is present in a concentration of from about 50 to about 200 millimolar.

9. Method for determining cholinesterase activity which comprises admixing a solution containing

cholinesterase with chromogenic and nonchromogenic substrates, buffer and chromogen and thereafter measuring the optical absorbance of the resulting admixture to determine the cholinesterase activity.

**10.** A method for determining enzyme activity without requiring predilution of the sample, which method consists essentially of admixing the sample with a chromogenic and nonchromogenic, hypochromogenic, bathochromogenic or hypsochromogenic substrate, buffer and chromogen and thereafter measuring the optical absorbance of the resulting material to determine the enzyme activity.

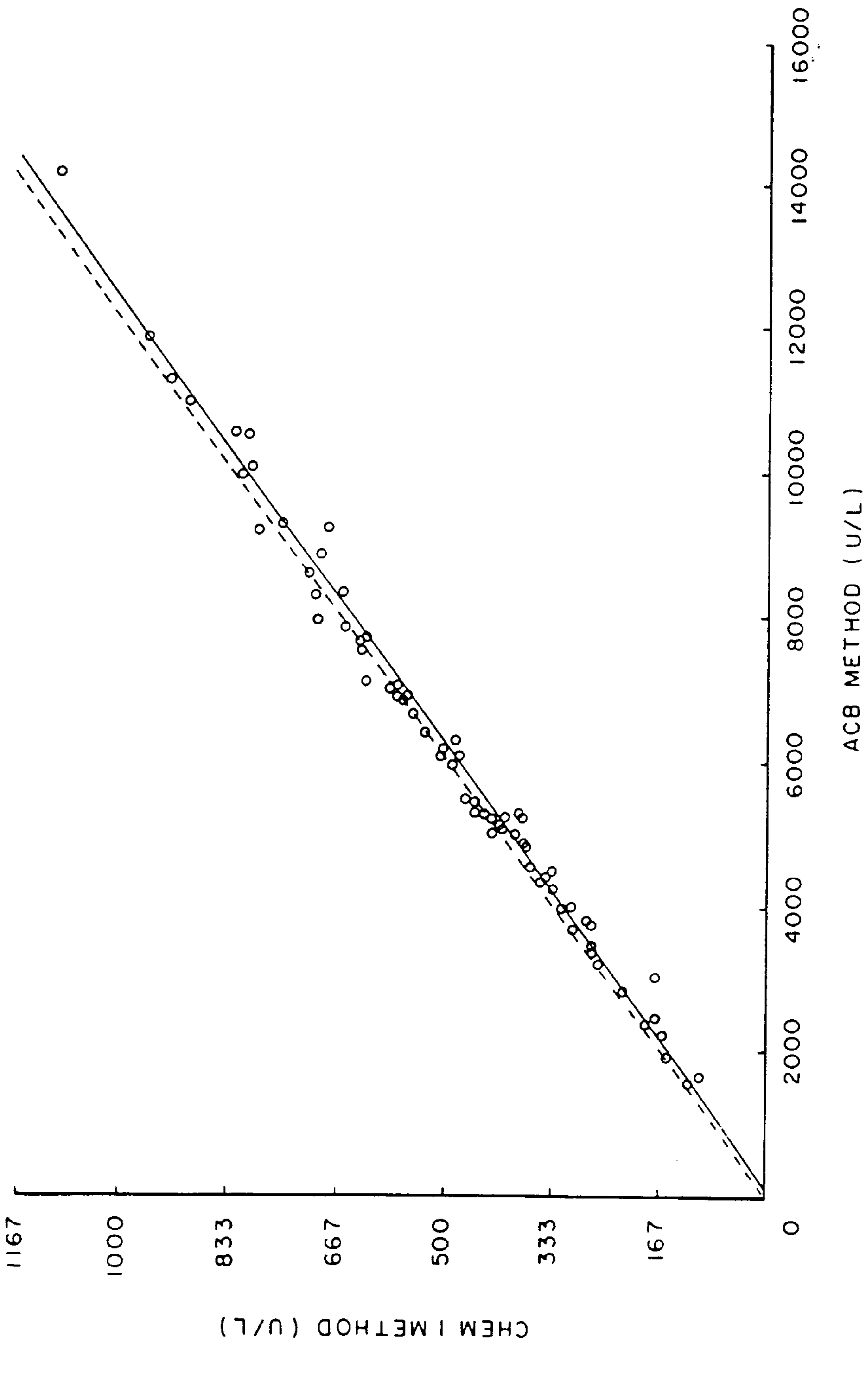
CHEM I METHOD (U/L)
1167
1000
833
667
500
333
167
0
2000
4000
6000
8000
10000
12000
14000
16000
ACB METHOD (U/L)

FIG. 1

European Patent Office

# EUROPEAN SEARCH REPORT

Application Number

**EP 91 10 9247**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | BIOCHIMICA ET BIOPHYSICA ACTA, vol. 480, no. 1, 11th January 1977, pages 137-142; E. REINER et al.: "Competition between substrates for acetylcholinesterase and cholinesterase"<br>* Whole document * | 1,2,5,9,10 | C 12 Q 1/00<br>C 12 Q 1/46 //<br>C 12 Q 1/37<br>C 12 Q 1/32<br>C 12 Q 1/34 |
| X | ARCHIVES OF BIOCHEMISTRY AND BIOPHYSICS, vol. 272, no. 1, July 1989, pages 52-68; R.C. BLAKE, II et al.: "The Michaelis constants of a nonchromogenic substrate may be determined using a chromogenic substrate"<br>* Abstract * | 1,10 | |
| X | EP-A-0 279 988 (ENZYMATICS, INC.)<br>* Abstract; column 1, lines 1-21; column 2, lines 9-42 * | 1,10 | |
| X | EP-A-0 144 798 (BOEHRINGER MANNHEIM GmbH)<br>* Pages 1-7 * | 1,10 | |
| X | ANALYTICAL LETTERS, vol. 13, no. B10, 1980, pages 851-860, Marcel Dekker, Inc.; B. MATTIASSON: "A simple method involving aqueous two-phase separation-systems for activity determination of enzymes hydrolyzing macro-molecular substrates"<br>* Abstract * | 1,10 | TECHNICAL FIELDS SEARCHED (Int. Cl.5)<br>C 12 Q |
| Y | IDEM | 1-10 | |
| X<br>Y | EP-A-0 327 952 (MILES INC.)<br>* Page 3, line 41 - page 4, line 36; page 6 * | 1,10<br>1,2,4-10 | |
| Y,D | ANNALS OF CLINICAL BIOCHEMISTRY, vol. 16, 1979, pages 57-75; E. SILK et al.: "Assay of cholinesterase in clinical chemistry"<br>* Page 65, column 2 - page 67, column 2 * | 1-10 | |

-/-

The present search report has been drawn up for all claims

| Place of search | Date of completion of search | Examiner |
|---|---|---|
| The Hague | 02 October 91 | HITCHEN C.E. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document

European
Patent Office

# EUROPEAN SEARCH REPORT

Application Number

EP 91 10 9247

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | CLINICAL CHEMISTRY, vol. 29, no. 6, 1983, pages 1065-1069; D.L. HAY et al.: "Rapid acetylcholinesterase screening test for neural tube defect"<br>* Whole document *<br>– – – | 1,2,4-10 | |
| A | GB-A-2 096 989 (KYOWA HAKKO KOGYO)<br>* Page 1, lines 1-64 *<br>– – – – – | 1-5 | |

TECHNICAL FIELDS SEARCHED (Int. Cl.5)

The present search report has been drawn up for all claims

| Place of search | Date of completion of search | Examiner |
|---|---|---|
| The Hague | 02 October 91 | HITCHEN C.E. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document